# EUROPEAN PATENT APPLICATION

(11) **EP 4 729 129 A2**
(43) Date of publication of application: **22.04.2026**
(21) Application number: 26156725.9
(22) Date of filing: 13.03.2020
(51) Int. Cl.: A61P 37/08

(54) **LIQUID COMPOSITION COMPRISING ANTIBODY OF HUMAN INTERLEUKIN-4 RECEPTOR ALPHA**

(30) Priority: 13.03.2019 CN 201910187179
(62) Divisional of application: 20770382.8
(71) Applicant: Connect Biopharma HongKong Limited, Kowloon (HK)
(72) Inventor: ZHENG, Wei, San Diego, CA 92130 (US); PAN, Wubin, Richmond, British Columbia (CA); YANG, Xin, Taicang 215400 (CN); ZHANG, Limin, Taicang 215400 (CN); JIANG, Jie, Taicang 215400 (CN)
(74) Representative: Schiweck Weinzierl Koch Patentanwälte Partnerschaft mbB

(57) **Abstract**

The present invention provides a liquid composition comprising an antibody of human interleukin-4 receptor alpha. The liquid composition comprises the antibody at a concentration of 50-200 mg/ml; and a buffer, a protective agent, and a surfactant as adjuvants. The liquid composition has a pH of 5.4-6.4.

## Description

The present application claims the priority benefit of Chinese Patent Application No. 201910187179.9, filed on 13 March 2019, which is hereby incorporated by reference in its entirety.

### TECHNICAL FIELD

The present invention relates to the field of biopharmaceutical formulation. Particularly, the present invention relates to a stable liquid formulation comprising an antibody at high concentration.

### BACKGROUND OF THE INVENTION

Human interleukin-4 receptor is known to produce a soluble form of protein (shIL-4Rα) that inhibits cell proliferation mediated by IL-4 and IL-5 up-regulation mediated by T cells. Two forms of the receptor are associated with allergic reaction, which manifests as diseases like allergic rhinitis, sinusitis, asthma, eczema, and the like. Therefore, a blocking antibody that targets the protein helps to treat and relieve such diseases.

Currently, monoclonal antibody medicaments targeting hIL-4R have entered clinical trials, such as Dupilumab, which has shown good efficacy in phase II clinical trial for the treatment of atopic dermatitis. However, for antibody medicaments, the best mode of administration is subcutaneous injection and relatively high doses are required to exert their effects, and accordingly high-concentration antibody formulations are generally required to be prepared. As is known in the prior art, the manufacture and application of a high-concentration antibody formulation are usually accompanied with many difficulties. For example, high viscosity of such a formulation may be difficult to be drawn and injected with a syringe, lead to large deviations in administration dosage due to high drug residue in a container or cartridge holding the formulation, cause pain at injection site, and the like. In addition, the high viscosity of the formulation may create serious process problems during manufacturing. For example, extremely high pressure may be needed during concentration and filtration steps, or even the formulation cannot pass through a filter membrane at all. Or, the high concentration antibody in such a formulation is prone to aggregate and form insoluble particles, thereby resulting in an instable formulation, increased immunogenicity, and more medication side effects, etc.

Therefore, there remains a need in the art to develop a novel antibody formulation targeting human interleukin-4 receptor which can meet manufacturing and clinical application requirements for high antibody concentration, long-term stability, no aggregation, and low viscosity, among the others.

### SUMMARY OF THE INVENTION

The purpose of the present invention is to provide a liquid composition comprising an antibody against human interleukin-4 receptor alpha and a formulation thereof, and such liquid composition and the formulation thereof can enable the antibody to be present stably at a high concentration and have a low viscosity.

Technical solutions provided by the present invention are as follows.

In one aspect, the present invention provides a liquid composition comprising an antibody against human interleukin-4 receptor alpha, wherein the liquid composition comprises the antibody at a concentration of 50-200 mg/ml; and a buffer, a protective agent and a surfactant and the like which serve as excipients, and the liquid composition has a pH of 5.4-6.4.

In the liquid composition, the antibody comprises a light chain variable region (VL) and a heavy chain variable region (VH), wherein the light chain variable region comprises CDR1 comprising the amino acid sequence as set forth in SEQ ID NO: 1, CDR2 comprising the amino acid sequence as set forth in SEQ ID NO: 2, and CDR3 comprising the amino acid sequence as set forth in SEQ ID NO: 3, and the heavy variable region comprises CDR1 comprising the amino acid sequence as set forth in SEQ ID NO: 5, CDR2 comprising the amino acid sequence as set forth in SEQ ID NO: 6, and CDR3 comprising the amino acid sequence as set forth in SEQ ID NO: 7.

Preferably, the antibody comprises a light chain variable region comprising the amino acid sequence as set forth in SEQ ID NO: 4, and a heavy variable region comprising the amino acid sequence as set forth in SEQ ID NO: 8.

According to specific embodiments of the present invention, the antibody has a kappa light chain constant region (CL) and a gamma heavy chain constant region (CH). Further preferably, the antibody comprises a light chain constant region comprising the amino acid sequence as set forth in SEQ ID NO: 9 and a heavy chain constant region comprising the amino acid sequence as set forth in SEQ ID NO: 10. More preferably, the antibody is a monoclonal antibody comprising two light chains and two heavy chains.
SEQ ID NO: 1 (LCDR1): RASQSVSSSYLA;
SEQ ID NO: 2 (LCDR2): GASSRAT;
SEQ ID NO: 3 (LCDR3): QQYDHSAGWT;
SEQ ID NO: 4 (VL): 
EIVLTQSPGTLSLSPGERATLSCRASQSVSSSYLAWYQQKPGQAPRLLIYGASSRAT
GIPDRFSGSGSGTDFTLTISRLEPEDFAVYYCQQYDHSAGWTFGQGTKVEIK.
SEQ ID NO: 5 (HCDR1): RNAMF;
SEQ ID NO: 6 (HCDR2): GIGTGGATSYADSVKGR;
SEQ ID NO: 7 (HCDR3): GRYYFDY;
SEQ ID NO: 8 (VH): 
EVQLVESGGGLVQPGGSLRLSCAASGFTFSRNAMFWVRQAPGKGLEWVSGIGTGG
ATSYADSVKGRFTISRDNAKNSLYLQMNSLRAEDTAVYYCARGRYYFDYWGQGTLVT
VSS.
SEQ ID NO: 9 (CL): 

.
SEQ ID NO: 10 (CH): 
ASTKGPSVFPLAPCSRSTSESTAALGCLVKDYFPEPVTVSWNSGALTSGVHTFPAVL
QSSGLYSLSSVVTVPSSSLGTKTYTCNVDHKPSNTKVDKRVESKYGPPCPPCPAPEF
LGGPSVFLFPPKPKDTLMISRTPEVTCVVVDVSQEDPEVQFNWYVDGVEVHNAKTK
PREEQFNSTYRVVSVLTVLHQDWLNGKEYKCKVSNKGLPSSIEKTISKAKGQPREPQ
VYTLPPSQEEMTKNQVSLTCLVKGFYPSDIAVEWESNGQPENNYKTTPPVLDSDGSF
FLYSRLTVDKSRWQEGNVFSCSVMHEALHNHYTQKSLSLSLG.
SEQ ID NO: 11 (L): 
EIVLTQSPGTLSLSPGERATLSCRASQSVSSSYLAWYQQKPGQAPRLLIYGASSRAT
GIPDRFSGSGSGTDFTLTISRLEPEDFAVYYCQQYDHSAGWTFGQGTKVEIKRTVAA
PSVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGEC.
SEQ ID NO: 12 (H):

EVQLVESGGGLVQPGGSLRLSCAASGFTFSRNAMFWVRQAPGKGLEWVSGIGTGG
ATSYADSVKGRFTISRDNAKNSLYLQMNSLRAEDTAVYYCARGRYYFDYWGQGTLVT
VSSASTKGPSVFPLAPCSRSTSESTAALGCLVKDYFPEPVTVSWNSGALTSGVHTFP
AVLQSSGLYSLSSVVTVPSSSLGTKTYTCNVDHKPSNTKVDKRVESKYGPPCPPCPA
PEFLGGPSVFLFPPKPKDTLMISRTPEVTCVVVDVSQEDPEVQFNWYVDGVEVHNA
KTKPREEQFNSTYRVVSVLTVLHQDWLNGKEYKCKVSNKGLPSSIEKTISKAKGQPR
EPQVYTLPPSQEEMTKNQVSLTCLVKGFYPSDIAVEWESNGQPENNYKTTPPVLDSD
GSFFLYSRLTVDKSRWQEGNVFSCSVMHEALHNHYTQKSLSLSLG.

Preferably, the antibody is present at a concentration of 100-200 mg/ml, more preferably 130-165 mg/ml, and further preferably 150±5 mg/ml.

In the liquid composition, the buffer is one or more selected from the group consisting of an acetate buffer, a phosphate buffer and an amino acid buffer, and the buffer is present at a concentration of 5-50 mmol/L; preferably, the buffer is an amino acid buffer at a concentration of 5-50 mmol/L.

Preferably, the acetate buffer is sodium acetate buffer, the phosphate buffer is sodium dihydrogen phosphate buffer, or the amino acid buffer is histidine hydrochloride buffer.

Preferably, the buffer is present at a concentration of 5-20 mmol/L.

Preferably, the buffer is histidine hydrochloride buffer at a concentration of 5-20 mmol/L, more preferably histidine hydrochloride buffer at a concentration of 10 mmol/L.

In the liquid composition, the protective agent is one or more selected from the group consisting of a sugar, an alcohol, an amino acid and a chloride salt, and the protective agent is present at a concentration of 40-220 mmol/L.

Preferably, the protective agent is one or more selected from the group consisting of a sugar, an alcohol and an amino acid, and the protective agent is present at a concentration of 40-220 mmol/L, and/or the protective agent is a chloride salt, and the protective agent is present at a concentration of 40-150 mmol/L.

Preferably, the sugar is trehalose and/or sucrose at a concentration of 40-150 mmol/L, more preferably 60-150 mmol/L; the alcohol is mannitol at a concentration of 40-220 mmol/L, more preferably 110-150 mmol/L; the amino acid is one or more selected from the group consisting of proline, arginine hydrochloride and glycine at a concentration of 40-220 mmol/L, more preferably 120-220 mmol/L; or the chloride salt is sodium chloride at a concentration of 40-150 mmol/L, more preferably 80-120 mmol/L.

More preferably, the protective agent is a combination of trehalose and sodium chloride; further preferably, the protective agent is a combination of 40-150 mmol/L trehalose and 40-150 mmol/L sodium chloride; still further preferably, the protective agent in the liquid composition is a combination of 60-150 mmol/L trehalose and 80-120 mmol/L sodium chloride, more preferably a combination of 60 mmol/L trehalose and 100 mmol/L sodium chloride.

In the liquid composition, the surfactant can be a non-ionic polymer, such as is one or more selected from the group consisting of Tween 80, Tween 20, Poloxamer and polyethylene glycol, and the surfactant is present at a concentration of 0.01%-0.2%.

Preferably, the surfactant is 0.01-0.03% Tween 80, more preferably 0.02% Tween 80.

The pharmaceutical composition provided by the present invention is a colorless to pale yellow transparent sterile solution with a slight opalescence. As detected, the liquid composition provided by the present invention has an osmotic pressure of 230-330 mOsmol/kg, a viscosity of < 30 cP, and a pH of 6.2±0.2.

According to specific embodiments of the present invention, the liquid composition is a formulation for injection, and preferably for subcutaneous or intravenous injection; and preferably the liquid composition is a formulation for subcutaneous or intravenous injection.

Preferably, the liquid composition comprises:
130-165 mg/ml, preferably 150±5 mg/ml of the antibody against human interleukin-4 receptor alpha;
10 mmol/L histidine hydrochloride;
60 mmol/L trehalose;
100 mmol/L sodium chloride;
0.02% Tween 80; and
the liquid composition has a pH of 6.2±0.2, preferably 6.2±0.05.

The formulation provided by the present invention is a colorless or pale yellow transparent sterile solution which has favorable long-term stability (it can be stored for 2 years at 2-8 °C and satisfy quality standards) and no aggregates (≤ 10.0%). The formulation of the present invention has a low viscosity (< 30 cP) and is characterized by a pH and an osmotic pressure (290-310 mOsmol/kg) suitable for subcutaneous injection. See Table 14 below for detail in this regard.

Concentrations mentioned above are all based on total volume or total weight of the liquid composition or the liquid formulation. In the context, terms "liquid formulation" and "liquid composition" can be used interchangeably.

According to specific embodiments of the present invention, the liquid composition is a formulation for subcutaneous injection, and additionally comprises sterile water for injection.

In another aspect, the present invention provides use of the liquid composition for manufacturing a medicament for the treatment of inflammation or allergic disease; preferably, the inflammation or allergic disease includes autoimmune disease, such as allergic dermatitis, asthma, eosinophilic esophagitis, eczema, allergic rhinitis, nasal polyp, rheumatoid arthritis, and the like.

In still another aspect, the present invention also provides other products related to the liquid composition.

The present invention provides a container, comprising the liquid composition of the present invention. For example, the container can be a 2 mL injection vial made of neutral borosilicate glass tubing, in which the fill volume of the liquid composition is greater than 1 mL per vial.

The present invention provides a kit, comprising the container provided by the present invention; and further comprising an instruction.

In yet another aspect, the present invention provides a method of preventing, treating or ameliorating inflammation or allergic disease, including administering to a subject in need thereof the liquid composition of the present invention. Preferably, the subject is a mammal, more preferably a human.

Preferably, the inflammation or allergic disease includes autoimmune disease, such as allergic dermatitis, asthma, eosinophilic esophagitis, eczema, allergic rhinitis, nasal polyp, rheumatoid arthritis, and the like.

The liquid composition provided by the present invention can be administrated to the subject by injection, for example by subcutaneous injection or intravenous injection.

Other medicaments can be used in combination with the liquid composition to prevent, treat or ameliorate inflammation or allergic disease. For example, the method further includes administering to the subject at least one medicament selected from the group consisting of an antiasthmatic such as albuterol etc., an antihistamine such as loratadine etc., an immunosuppressive agent such as tacrolimus and pimecrolimus etc., an M receptor blocker such as ipratropium bromide etc., a leukotriene receptor blocker such as montelukast etc., a phosphodiesterase inhibitor such as theophylline etc., a non-steroidal anti-inflammatory drug such as 5-aminosalicylic acid etc., and a hormone such as beclomethasone and budesonide etc. Preferably, the medicament(s) and the liquid composition of the present invention are administrated simultaneously or sequentially.

The inventors of the present invention successfully developed a novel liquid composition for the antibody against human interleukin-4 receptor alpha, which provides a basis for the manufacture of a medicament. The liquid composition provided by the present invention contains a high concentration of the antibody against human interleukin-4 receptor alpha, and when administrated subcutaneously or intravenously, it can satisfy medication requirements and improve therapeutic effects through providing a high dose of the antibody. Meanwhile, even a high concentration of the antibody is comprised, the present liquid composition exhibits no aggregation of the antibody and meanwhile has a fairly low viscosity, which makes it possible to deliver the composition through a fine needle and a needle tube easily and thereby minimize the discomfort of patients. The liquid composition of the invention also has the advantages of easy manufacture and storage. In addition, the liquid composition has sufficient physical and chemical stabilities, in which the contents of insoluble particles are within the ranges prescribed in the China Pharmacopoeia (the number of insoluble particles with a particle size of ≥ 10 µm is ≤ 6000/vial, and the number of insoluble particles with a particle size of ≥ 25 µm is ≤ 600/vial). The liquid composition can also be frozen and thawed repeatedly, is resistant to shaking, has good thermal stability, and meets the requirements for manufacturing of a medicament.

The liquid composition provided by the present invention was assessed for its binding ability with human interleukin-4 receptor alpha and biological activity of blocking STAT-6 signal transduction. The results show that the liquid composition provided by the invention can bind with the antigen IL-4Rα stably and effectively, and block STAT-6 signal transduction effectively.

### BRIEF DESCRIPTION OF THE DRAWINGS

Embodiments of the present invention are described in detail hereinafter in combination with the accompanying drawings, in which:
Figures 1 and 2 show results of SEC purity of the formulations examined during pH screening.
Figures 3 and 4 show results of nrCE-SDS purity of the formulations examined during pH screening.
Figures 5 and 6 show results of rCE-SDS purity of the formulations examined during pH screening.
Figures 7 and 8 show results of changes in CEX neutral peak of the formulations examined during pH screening respectively.
Figures 9 and 10 show results of SEC purity of the formulations examined during protective agent screening respectively.
Figures 11 and 12 show results of nrCE-SDS purity of the formulations examined during protective agent screening respectively.
Figures 13 and 14 show results of rCE-SDS purity of the formulations examined during protective agent screening respectively.
Figures 15 and 16 show results of changes in CEX neutral peak of the formulations examined during protective agent screening respectively.
Figure 17 shows comparison results of viscosity of the formulations examined during protective agent screening.
Figure 18 shows results of viscosity of the formulations at different concentrations examined without protective agent.
Figure 19 shows results of biological activity detection of CBP-201.

### DETAILED DESCRIPTION OF PREFERRED EMBODIMENTS

The present invention will be further described in detail in combination with the particular embodiments hereinafter. It will be appreciated by those skilled in the art that the embodiments provided are only used to illustrate the present invention, rather than limiting the scope of the present invention in any way.

Experimental methods in the following Examples are all conventional methods, unless particularly stated. Raw materials and reagents used in the following Examples are all products that commercially available, unless particularly stated.

The antibody expressed as "CBP-201" in the following Examples is a monoclonal antibody comprising a light chain variable region as set forth in SEQ ID NO: 4, and a heavy variable region as set forth in SEQ ID NO: 8; a light chain constant region as set forth in SEQ ID NO: 9 and a heavy chain constant region as set forth in SEQ ID NO: 10; or a light chain as set forth in SEQ ID NO: 11 and a heavy chain as set forth in SEQ ID NO: 12. In the context, terms "CBP-201" and "protein" can be used interchangeably. In addition, terms "CBP-201 formulation" and "CBP-201 liquid composition" can also be used interchangeably.

General methods used in following Examples include:
(1) Determination of pH value:
   The determination of pH value is conducted with reference to the pH measuring method described in General Rule 0631, Volume IV, Pharmacopoeia of the People's Republic of China (2015 edition).
(2) Accelerated stability test at 40 °C:
   Research on stability is conducted by storing a test sample of a formulation prepared under high temperature and high humidity (40 °C±2 °C / 75±5% RH) for 2 weeks or 4 weeks for examining the stability of the formulation.
(3) Detection of protein concentration:
   The protein concentration is detected by an UV spectrophotometer using extinction coefficient (ε). According to the Lambert-Beer law, the absorbance value of a sample is calculated according to the formula: A=ε• C • L/N, in which "C" represents the concentration of the protein in the sample, mg/mL; "L" represents optical path which is 1 cm; "A" represents the absorbance value; "ε" represents the extinction coefficient; and "N" represents the dilution ratio of the sample. In this regard, the protein concentration in the sample is calculated according to the formula: C=A/ε×N.

The theoretical extinction coefficient of the protein (the antibody) can be calculated according to the following formula: mass extinction coefficient ε = (5500nw + 1490ny + 125nc) • M⁻¹• cm⁻¹, in which "nw" represents the number of Trp in the amino acid sequence of the protein; "ny" represents the number of Tyr in the amino acid sequence of the protein; "nc" represents the number of Cys in the amino acid sequence of the protein; "M" represents the molecular weight of the protein, and "cm" represents optical distance.

According to the sequences of the CBP-201 antibody, the mass extinction coefficient ε is calculated to be 1.46. The absorbance value A of the sample at 280 nm is measured by an UV spectrophotometer, and accordingly the antibody concentration is calculated.

### (4) Purity detection by SEC:

Size exclusion chromatography (SEC-HPLC) with following chromatographic conditions is used:
Column: TSKgel G3000SW_{XL} 7.8*300 mm column;
Column temperature: room temperature;
Detector: DAD detector;
Detective wavelength: 280 nm;
Flow rate: 0.7 mL/min;
Sample dilution: diluted with ultrapure water to 5.0 mg/mL;
Injection volume: 10 µl;
Mobile phase: 25 mM phosphate (pH 6.8 ± 0.1) and 0.3 M sodium chloride;
Elution mode: gradient elution

| Time (min) | D % | Flow rate (mL/min) |
|---|---|---|
| 0 | 100 | 0.7 |
| 25 | 100 | 0.7 |

Detection: the peak area normalization method is used for calculating peak area percentages of the main peak, and peaks of HMW and LMW components.

### (5) Purity detection by nrCE-SDS:

The purity of CBP-201 is quantitatively determined based on molecular weights under non-reducing conditions.

Detection: according to the area normalization method, the purity of the main peak is calculated as the percentage of the corrected peak area of IgG main peak to the sum of all corrected peak areas.

### (6) Purity detection by rCE-SDS:

The purity of CBP-201 is quantitatively determined based on molecular weights under reducing conditions.

Detection: according to the area normalization method, purity of each of LC, NGHC and HC (i.e. CAP) is calculated respectively as the percentage of the corrected peak area of each of LC, NGHC and HC to the sum of all corrected peak areas. The purity of a sample is the sum of the purities of LC and HC.

### (7) Charge heterogeneity detection (CEX neutral peak):

The detection is conducted with reference to the ion chromatography as described in General Rule 0514, Volume III, Pharmacopoeia of the People's Republic of China (2015 edition). The column used is BiomAb NP5, PK, 4.6×250 mm available from Agilent; chromatographic peak integration results are evaluated with reference to a reference, and the highest peak is the main peak, peaks integrated earlier than the retention time of the main peak are defined as acid peaks, and peaks integrated later than the retention time of the main peak are defined as alkaline peaks.

### (8) DSC detection:

DSC thermal analysis, also referred to differential scanning calorimeter, is a technique for recording the endothermic or exothermic rate of a sample by a differential scanning calorimeter, and using heat flux dH/dt (in millijoules/second) as the ordinate and temperature T or time t as the abscissa, to measure the phase-transition temperature of the sample, and then the stability of the sample can be judged.

### (9) Viscosity detection:

The detection is conducted using DV2T Viscometer available from Brook Field and with reference to the viscosity determination method-the third method (Rotational viscosity measurement method) described in General Rule 0633, Volume IV, Pharmacopoeia of the People's Republic of China (2015 edition).

### (10) Visible foreign matter detection:

The detection is conducted with reference to the method for examining visible foreign matter-the first method (lamp test) described in General Rule 0904, Volume IV, Pharmacopoeia of the People's Republic of China (2015 edition).

### (11) Particle size detection:

The detection is conducted with reference to the insoluble particle inspection method -the first method (Light Blockage) described in General Rule 0903, Volume IV, Pharmacopoeia of the People's Republic of China (2015 edition).

### Example 1 pH and buffer

In this example, six pH values, i.e. 5.4, 5.6, 5.8, 6.0, 6.2 and 6.4, and a buffer selected from sodium acetate, histidine hydrochloride and sodium dihydrogen phosphate were used for studying pH ranges and buffers for the CBP-201 formulation. Sodium chloride was additionally added into each sample. Specifically, in an accelerated test at 40 °C, following protein solutions containing 133.6 mg/ml CBP-201 were examined in order to determine suitable pH range and buffer (see Table 1).

**Table 1: pH and buffer screening solutions**

| Number | Protein solution | pH | Volume prepared |
|---|---|---|---|
| A | 10 mM NaAc, 150 mM NaCl | 5.4 | 1L |
| B | 10 mM NaAc, 150 mM NaCl | 5.6 | 1L |
| C | 10 mM His-HCl, 150 mM NaCl | 5.8 | 3L |
| D | 10 mM His-HCl, 150 mM NaCl | 6.5 | 1L |
| E | 10 mM NaH₂PO₄, 150 mM NaCl | 6.4 | 1L |

Through the accelerated stability test at 40 °C, the protein solutions were tested for appearance, protein concentration, SEC purity, CE-SDS purity, charge heterogeneity, DSC and viscosity.

Results of the appearance inspection: all protein solutions were whitish in appearance, and no visible foreign matters were observed.

Results of the protein concentration: the protein concentrations of the protein solutions were all in a range of 133.6±5% mg/ml, and no obvious increase or decrease in concentration was observed.

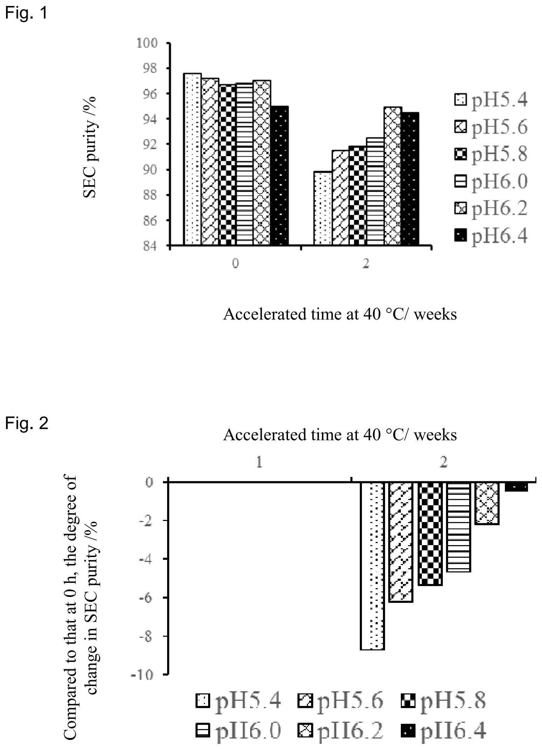

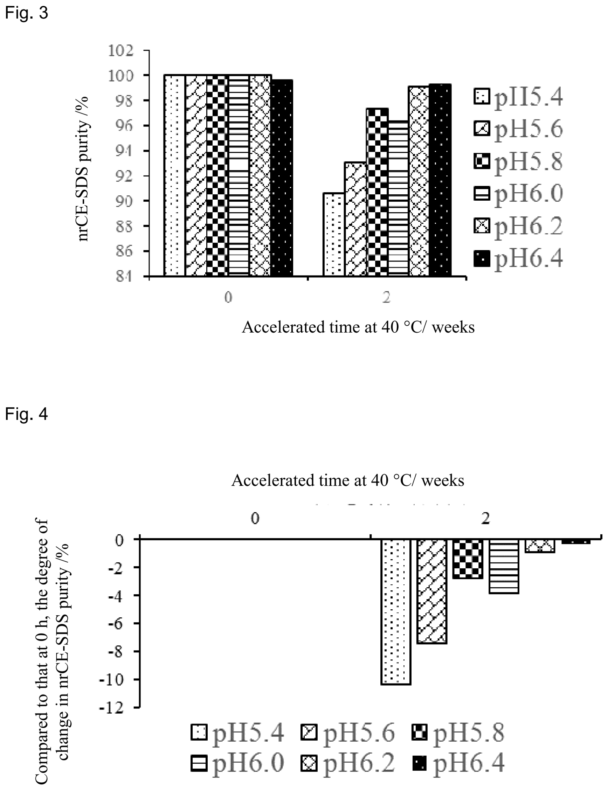

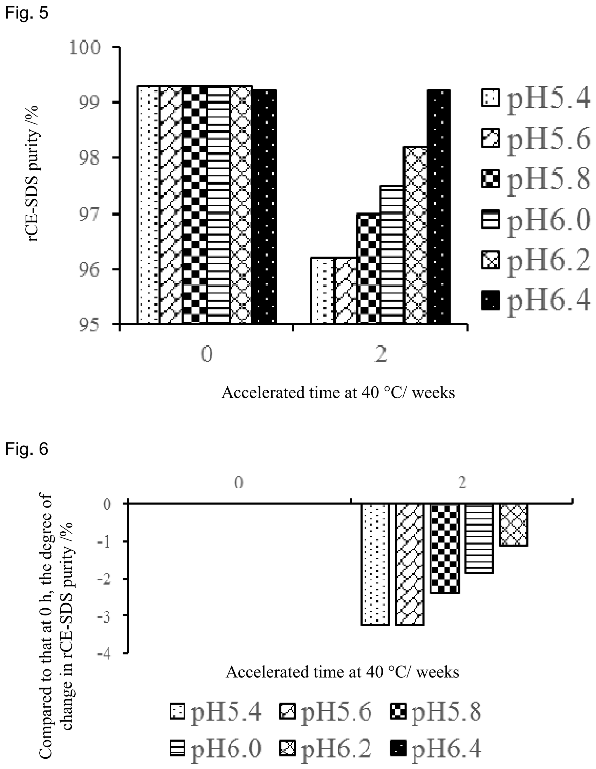

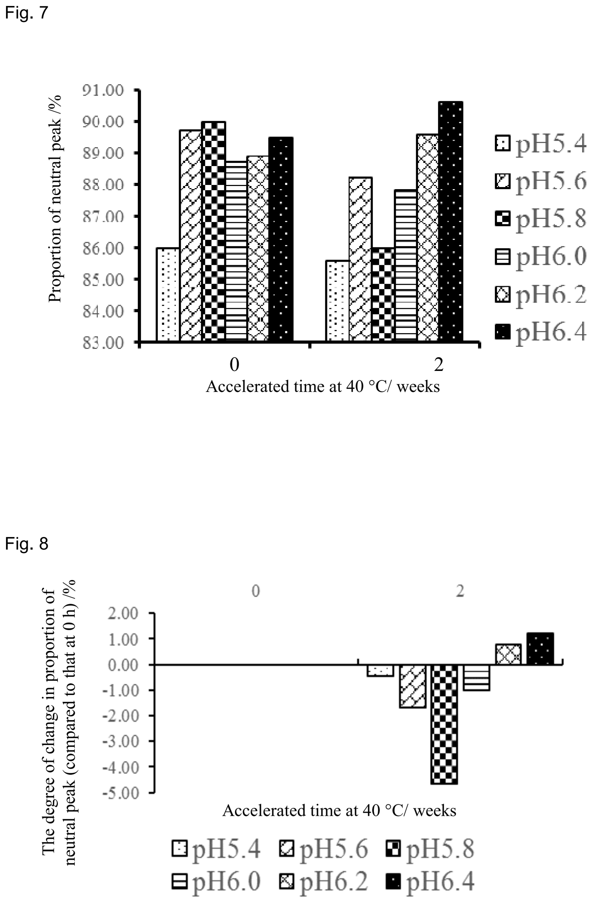

In addition, after acceleration at 40 °C for 2 weeks, following results were obtained from the protein solutions at different pH values:
1. Results of SEC purity were shown in Figures 1-2. After a 2 week acceleration, the absolute values of SEC purity of the tested solutions were ranked as follows: the solution at pH 6.2 > the solution at pH 6.4 > the solution at pH 6.0 > the solution at pH 5.8 > the solution at pH 5.6 > the solution at pH 5.4 (Figure 1). Additionally, the degrees of decrease in SEC purity of the tested solutions (compared to that at 0 h respectively) were ranked as follows: the solution at pH 5.4 > the solution at pH 5.6 > the solution at pH 5.8 > the solution at pH 6.0 > the solution at pH 6.2 > the solution at pH 6.4 (Figure 2).
2. Results of nrCE-SDS were shown in Figures 3-4. After a 2 week acceleration, the absolute values of nrCE-SDS purity of the tested solutions were ranked as follows: the solution at pH 6.4 > the solution at pH 6.2> the solution at pH 5.8 > the solution at pH 6.0 > the solution at pH 5.6 > the solution at pH 5.4 (Figure 3). Additionally, the degrees of decrease in nrCE-SDS purity of the tested solutions (compared to that at 0 h respectively) were ranked as follows: the solution at pH 5.4 > the solution at pH 5.6 > the solution at pH 6.0 > the solution at pH 5.8 > the solution at pH 6.2 > the solution at pH 6.4 (Figure 4). Further, the nrCE-SDS purity of the solution at pH 6.0 was 97.5% following a 2 week accelerated storage, which is a rather high purity. The nrCE-SDS purities of the solutions at pH 6.2 and 6.4 were higher than that at pH 6.0.
3. Results of rCE-SDS were shown in Figures 5-6. After a 2 week acceleration, the absolute values of rCE-SDS purity of the tested solutions were ranked as follows: the solution at pH 6.4 > the solution at pH 6.2 > the solution at pH 6.0 > the solution at pH 5.8 > the solution at pH 5.6 = the solution at pH 5.4 (Figure 5). Additionally, the degrees of decrease in rCE-SDS purity of the tested solutions were ranked as follows: the solution at pH 5.4 = the solution at pH 5.6 > the solution at pH 5.8 > the solution at pH 6.0 > the solution at pH 6.2 > the solution at pH 6.4 (Figure 6).
4. Results of changes in CEX neutral peak were shown in Figures 7-8. After a 2 week acceleration, the proportion of CEX neutral peak of the tested solutions were ranked as follows: the solution at pH 6.4 > the solution at pH 6.2 > the solution at pH 5.6 > the solution at pH 6.0 > the solution at pH 5.8 > the solution at pH 5.4 (Figure 7). Additionally, the degrees of change (decrease) in the proportion of CEX neutral peak of the tested solutions (compared to that at 0 h respectively) following a 2 week accelerated storage were ranked as follows : the solution at pH 5.8 > the solution at pH 5.6 > the solution at pH 5.4 > the solution at pH 6.0 > the solution at pH 6.2 > the solution at pH 6.4 (Figure 8).
5. DSC results:
   The proteins in the 6 solutions having different pH values and buffers as shown in Table 1 were all stable, and there was no significant difference between them.
6. Results of viscosity:
   Comparison between viscosities of the tested protein solutions having different buffers showed that there was a small difference in viscosity between the solutions having His-HCl buffer system and the solution having phosphate buffer system, and viscosities of all those solutions were lower than that of the solution having acetate buffer system. For the solutions having His-HCl buffer system, their viscosities were in a range of 8.7±0.8 cP, which were lower than the expected 20 cP, and the solution having a higher pH showed a lower viscosity.

Based on the results obtained from the detection of SEC, nrCE-SDS, rCE-SDS, CEX, DSC and viscosity as above, it can be concluded that a higher pH has a better stabilizing effect on the protein, and according to their stabilization effects, the pH values can be ranked as follows: pH 6.4 > pH 6.2 > pH 6.0. That is, the protein can be stabilized well under an acid-base condition of pH 6.2±0.2.

### Example 2 Protective agents

In this example, protective agents suitable for the CBP-201 formulation were studied. Protein solutions which have a pH of 6.0 and trehalose, sucrose, mannitol, proline, arginine hydrochloride, glycine or sodium chloride added were prepared, in which the solution with sodium chloride added was used as a control. Specifically, in an accelerated stability test at 40 °C, following protein solutions containing 133.6 mg/ml CBP-201 were examined in order to determine suitable protective agent (see Table 2).

**Table 2: Protective agents**

| Number | Protein solution |
|---|---|
| A | pH 6.0, 10 mmol/L His-HCl, 0.13 M Suc |
| B | pH 6.0, 10 mmol/L His-HCl, 0.13 M Tre |
| C | pH 6.0, 10 mmol/L His-HCl, 0.13 M Man |
| D | pH 6.0, 10 mmol/L His-HCl, 0.22 M Pro |
| E | pH 6.0, 10 mM His-HCl, 0.128 M Arg |
| F | pH 6.0, 10 mmol/L His-HCl, 0.22 M Gly |
| G | pH 6.0, 10 mmol/L His-HCl, 0.12 M NaCl |

Through the accelerated stability test at 40 °C, the protein solutions were tested for appearance, protein concentration, SEC purity, CE-SDS purity, charge heterogeneity, viscosity and DSC.

Results of the appearance inspection: all protein solutions were whitish in appearance.

Results of the protein concentration: the protein concentrations of the protein solutions were all in a range of 133.6±5% mg/ml.

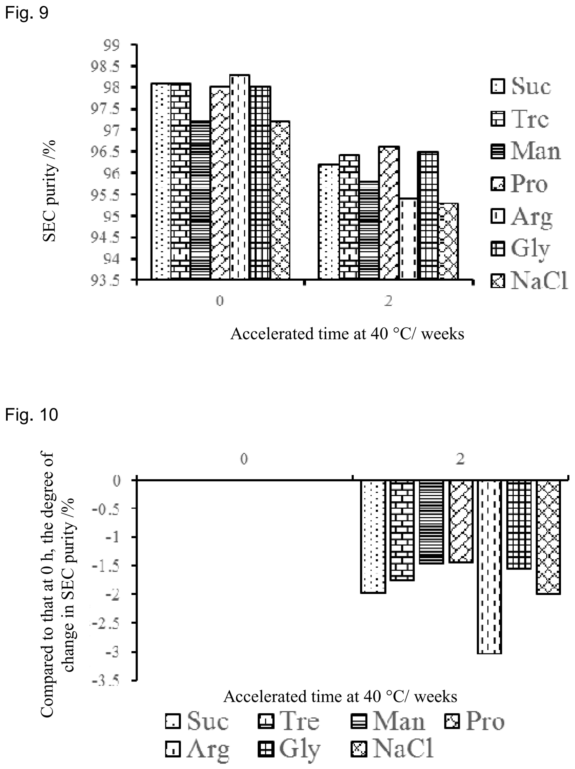

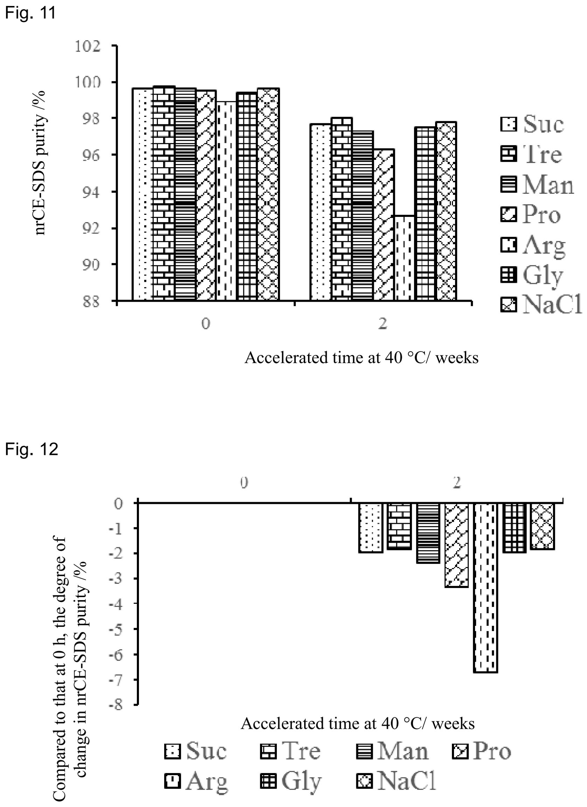

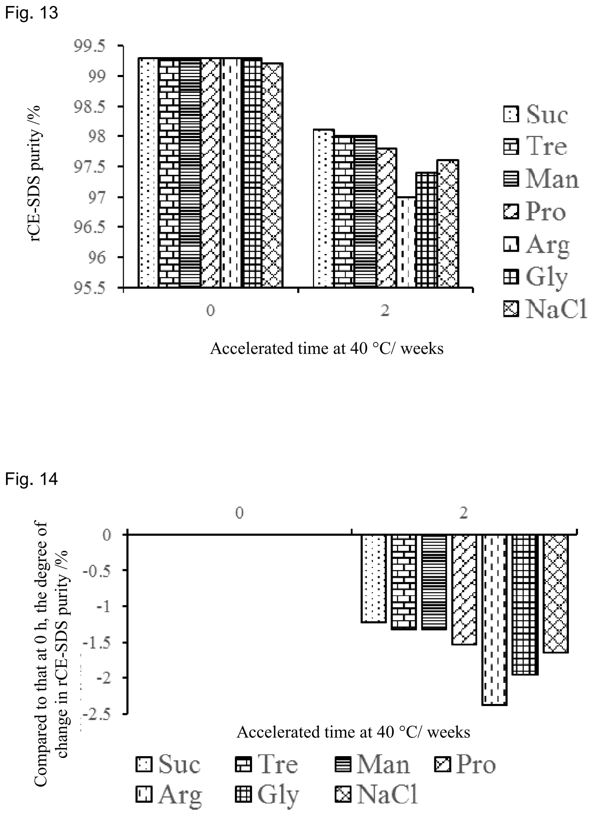

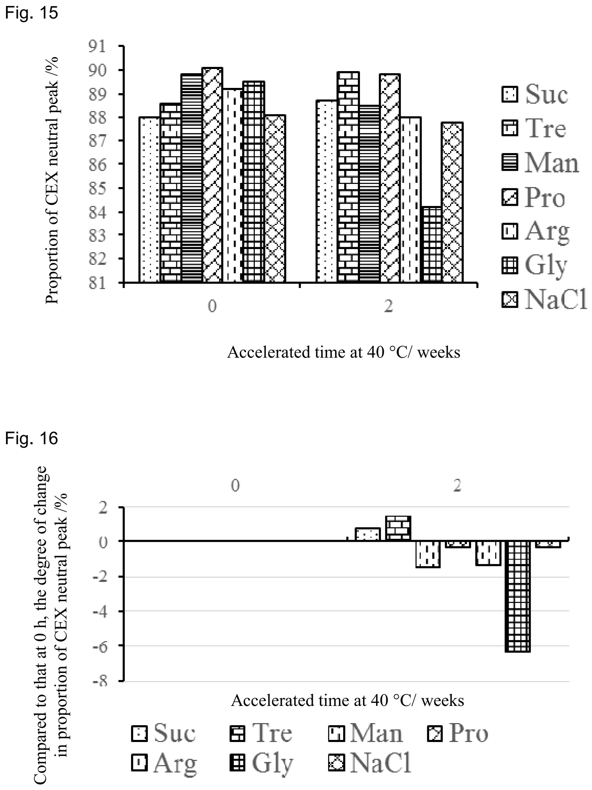

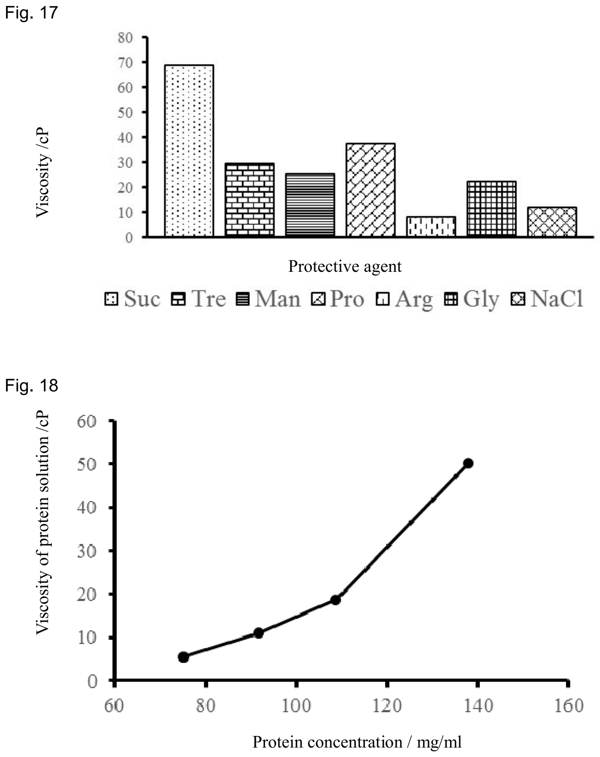

In addition, after acceleration at 40 °C for 2 weeks, following results were obtained from the protein solutions with different protective agents:
1. Results of SEC purity were shown in Figures 9-10. After a 2 week acceleration, the absolute values of SEC purity of the tested solutions were ranked as follows: the solution with Pro > the solution with Gly > the solution with Tre > the solution with Suc > the solution with Man > the solution with Arg > the solution with NaCl, in which the protein solution with NaCl as the protective agent had the lowest SEC purity of 95.3%, which however was acceptable (Figure 9). Additionally, the degrees of decrease in SEC purity of the tested solutions (compared to that at 0 h respectively) were ranked as follows: the solution with Arg > the solution with NaCl > the solution with Suc > the solution with Tre > the solution with Gly > the solution with Pro > the solution with Man. Even the protein solution with Arg as the protective agent had the largest degree of decrease in SEC purity, i.e. 3.04%, it still had an acceptable SEC purity of 95.4% following a 2 week accelerated storage (Figure 10).
2. Results of nrCE-SDS were shown in Figures 11-12. After a 2 week acceleration, the nrCE-SDS purities of the tested solutions were ranked as follows: the solution with Tre > the solution with NaCl > the solution with Suc > the solution with Gly > the solution with Man > the solution with Pro > the solution with Arg (Figure 11). Additionally, the degrees of decrease in nrCE-SDS purity of the tested solutions (compared to that at 0 h respectively) were ranked as follows: the solution with Arg > the solution with Pro > the solution with Man > the solution with Gly > the solution with Suc > the solution with NaCl > the solution with Tre (Figure 12). It could be seen, the nrCE-SDS purities which exhibited a small degree of decrease compared to that at 0h respectively and maintained at a relatively high level after a 2 week acceleration at 40 °C were ranked as follows: the solution with Tre > the solution with NaCl > the solution with Suc > the solution with Gly > the solution with Man > the solution with Pro > the solution with Arg.
3. Results of rCE-SDS were shown in Figures 13-14. After a 2 week acceleration, the absolute values of rCE-SDS purity of the tested solutions were ranked as follows: the solution with Suc > the solution with Man = the solution with Tre > the solution with Pro > the solution with NaCl > the solution with Gly > the solution with Arg (Figure 13). Additionally, compared to that at 0 h respectively, the degrees of decrease in rCE-SDS purity of the tested solutions were ranked as follows: the solution with Arg > the solution with Gly > the solution with NaCl > the solution with Pro > the solution with Tre = the solution with Man > the solution with Suc (Figure 14). It could be seen, the rCE-SDS purities which exhibited a small degree of decrease compared to that at 0h respectively and maintained at a relatively high level after a 2 week acceleration at 40 °C were ranked as follows: the solution with Suc > the solution with Man = the solution with Tre > the solution with Pro > the solution with NaCl > the solution with Gly > the solution with Arg.
4. Results of changes in CEX neutral peak were shown in Figures 15-16. After a 2 week acceleration, the proportions of CEX neutral peak of the tested solutions were ranked as follows: the solution with Tre > the solution with Pro > the solution with Suc > the solution with Man > the solution with Arg > the solution with NaCl > the solution with Gly (Figure 15). Additionally, the degrees of change (decrease) in proportion of CEX neutral peak of the tested solutions (compared to that at 0 h respectively) following a 2 week accelerated storage were ranked as follows: the solution with Gly > the solution with Man > the solution with Arg > the solution with NaCl > the solution with Pro > the solution with Suc (increase) > Tre (increase) (Figure 16). It can be concluded from the results that Tre occupies first place with regard to keeping charges stable, Suc comes the second, followed by Pro, Man, Arg, NaCl, Gly sequentially.
5. Results of viscosity were shown in Figure 17. After adding different protective agents, the viscosities of the protein solutions were ranked as follows: the solution with Suc > the solution with Pro > the solution with Tre > the solution with Man > the solution with Gly > the solution with NaCl > the solution with Arg.
6. DSC results: for the solutions comprising the 7 protective agents respectively, Tm1 and Tm2 values were all acceptable and the proteins therein were structurally stable, and there was no significant difference between them.

### Example 3 Viscosity evaluation on protein solutions with buffer only

This example was conducted to explore the change of viscosity with concentration of CBP-201 formulations with buffer (10 mmol/L His-HCl, pH 6.0) only (no protective agent added), to assess the effect of protective agent in reducing the viscosity of the formulations. A formulation with the maximum concentration was prepared and viscosity was measured, to provide a basis for the selection of protein concentration in the formulation. In this example, formulations were prepared without NaCl and compared with the formulation containing NaCl in the above example.

The protein was dialyzed into a dialysis buffer (10 mmol/L His-HCl, pH 6.0), and the dialyzed protein was concentrated to concentrations of 71.23 mg/ml, 89.04 mg/ml, 106.85 mg/ml, 133.56 mg/ml, and >151.37 mg/ml respectively (protein concentration detected), and then the formulations were filtrated. Appearance and viscosity were detected. Results obtained were shown in Table 3 and Figure 18.

**Table 3: Detection results of the appearance and the viscosity of the CBP-201 formulations**

| Number | Protein concentration (mg/ml) | Appearance | Viscosity (cP) / temperature (°C) |
|---|---|---|---|
| 1 | 75.15 | Whitish, no visible foreign matter | 5.51/25.0 |
| 2 | 91.64 | whitish, no visible foreign matter | 11.01/24.8 |
| 3 | 108.79 | whitish, no visible foreign matter | 18.80/24.8 |
| 4 | 137.94 | whitish, no visible foreign matter | 50.33/24.7 |
| 5 | 164.08 | Whitish, no visible foreign matter | The filtered protein solution was less than 0.5 mL, and viscosity was not detected |

The results showed that the viscosity of the protein solution increased with the increase of protein concentration. The viscosity of the protein solution having a target concentration of 133.6 mg/ml (the actual concentration was 137.94 mg/ml) was 50.33 cP, and only the protein solution containing Suc in the screening for suitable protective agent in Example 2 had a viscosity (68.75 cP) larger than that value. In other words, among the seven protective agent alternatives, only Suc increased the viscosity of the protein solution, and other protective agents reduced the viscosity of protein solutions to different levels. Therefore, the protective agents preferentially selected with respect to the effects on reducing the viscosity of the formulation can be ranked as follows: Arg, NaCl, Gly, Man, Tre and Pro.

### Example 4: Combination of components in formulation

Based on the results obtained from the screening tests, a study on a combination of components in the CBP-201 formulation was conducted. Compositions of the protein solutions studied were shown in Table 4.

**Table 4: Combinations of the components**

| Number | pH | His-HCl (mmol/L) | Tre (mmol/L) | NaCl (mmol/L) | Arg-HCl (mmol/L) | Tween 80 (w/v, %) |
|---|---|---|---|---|---|---|
| 1 | 6.2 | 10 | 60 | 100 | - | 0.02 |
| 2 | 6.2 | 10 | 130 | 50 | - | 0.02 |
| 3 | 6.2 | 10 | 150 | 40 | - | - |
| 4 | 6.2 | 10 | 150 | 40 | - | 0.02 |
| 5 | 6.2 | 10 | 150 | 40 | - | 0.2 |
| 6 | 6.2 | 10 | 150 | - | 46 | 0.02 |
| 7 | 6.0 | 10 | 150 | 40 | - | 0.02 |
| 8 | 6.0 | 10 | - | 150 | - | - |
| 9 | 6.4 | 10 | 150 | 40 | - | 0.02 |

The protein solutions contain 133.6 mg/ml CBP-201 and were examined through accelerated stability tests at 4 °C and 40 °C. Through the accelerated stability tests at 4 °C and 40 °C, the solutions were detected for visible foreign matter, particular size (MFI/FLOWCAM), protein concentration, SEC purity, DSC, osmotic pressure, viscosity, CE-SDS and CEX and results obtained were shown in Tables 5-11.

**Table 5: Results of the appearance inspection**

| Number | Appearance description | | | |
|---|---|---|---|---|
| | 0 h | 4 °C 2 W | 40 °C 2 W | 40 °C 4 W |
| 1 | whitish, no visible foreign matter | whitish, no visible foreign matter | whitish, no visible foreign matter | whitish, no visible foreign matter |
| 2 | whitish, no visible foreign matter | whitish, no visible foreign matter | whitish, no visible foreign matter | whitish, no visible foreign matter |
| 3 | whitish, no visible foreign matter | whitish, no visible foreign matter | whitish, no visible foreign matter | whitish, no visible foreign matter |
| 4 | whitish, no visible foreign matter | whitish, no visible foreign matter | whitish, no visible foreign matter | whitish, no visible foreign matter |
| 5 | whitish, no visible foreign matter | whitish, no visible foreign matter | whitish, no visible foreign matter | whitish, no visible foreign matter |
| 6 | whitish, no visible foreign matter | whitish, no visible foreign matter | whitish, no visible foreign matter | whitish, no visible foreign matter |
| 7 | whitish, no visible foreign matter | whitish, no visible foreign matter | whitish, no visible foreign matter | whitish, no visible foreign matter |
| 8 | whitish, no visible foreign matter | whitish, no visible foreign matter | whitish, no visible foreign matter | whitish, no visible foreign matter |
| 9 | whitish, no visible foreign matter | whitish, no visible foreign matter | whitish, no visible foreign matter | whitish, no visible foreign matter |

**Table 6: Detection results of the protein concentration**

| Number | Protein concentration (mg/ml) | | | |
|---|---|---|---|---|
| | 0 h | 4 °C 2 W | 40 °C 2 W | 40 °C 4 W |
| 1 | 139.97 | 134.05 | 135.59 | 138.93 |
| 2 | 139.51 | 132.49 | 137.18 | 130.11 |
| 3 | 139.56 | 141.48 | 134.85 | 127.78 |
| 4 | 135.21 | 130.58 | 139.75 | 136.38 |
| 5 | 138.33 | 136.71 | 131.56 | 134.96 |
| 6 | 136.38 | 140.82 | 134.41 | 139.64 |
| 7 | 129.56 | 137.26 | 134.91 | 139.01 |
| 8 | 139.70 | 130.22 | 139.39 | 133.72 |
| 9 | 139.81 | 133.31 | 130.61 | 138.55 |

Results of the appearance and the protein concentration showed that after being stored at 4 °C for 2 weeks and 40 °C for 4 weeks, no significant decrease in protein concentration was observed for the protein solutions having the combinations of components numbered 1 to 9, and the protein solutions were all whitish in appearance, which is related to the high protein concentrations of the solutions and the property of the protein per se. In this regard, the combinations of components numbered 1 to 9 were all acceptable based on the results of the appearances and the protein concentrations.

**Table 7: SEC purity**

| Number | SEC purity (%) | | | |
|---|---|---|---|---|
| | 0 h | 4 °C 2 W | 40 °C 2 W | 40 °C 4 W |
| 1 | 98.7 | 98.5 | 97.9 | 95.3; HMW=2.0; LMW=2.6 |
| 2 | 97.4 | 96.8 | 97.3 | 94.8; HMW=2.0; LMW=3.2 |
| 3 | 98.1 | 97.0 | 97.5 | 95.0; HMW=2.1; LMW=2.9 |
| 4 | 98.5 | 97.1 | 97.3 | 95.0; HMW=2.1; LMW=2.9 |
| 5 | 98.2 | 97.6 | 95.3 | 92.8; HMW=4.3; LMW=2.9 |
| 6 | 97.3 | 97.4 | 97.5 | 96.6; HMW=1.5; LMW=2.0 |
| 7 | 97.2 | 97.7 | 97.4 | 94.8; HMW=1.8; LMW=3.3 |
| 8 | 97.2 | 97.8 | 94.5 | 85.6; HMW=1.8; LMW=12.2 |
| 9 | 97.5 | 98.1 | 97.4 | 95.4; HMW=2.6; LMW=2.0 |

The data in Table 7 showed that the protein solution having the combination of components numbered 2 had the lowest but acceptable SEC purity of 96.8% after being stored at 4 °C for 2 weeks. Additionally, compared to the SEC purity at 0 h, the largest degree of decrease in SEC purity was observed for the protein solution having the combination of components numbered 4 and the degree of decrease was 1.44%, but the decreased SEC purity (97.1%) was still acceptable. In this regard, all solutions having the combinations of components numbered 1 to 9 had acceptable SEC purities after being stored for two weeks at 4 °C.

After being stored at 40 °C for 4 weeks, the protein solutions having the combinations of components numbered 5 and 8 showed significant decreases in SEC purity (compared to that at 0 h respectively) and thus had low SEC purities which were not acceptable. SEC purities of the protein solutions having other different combinations of components after being stored at 40°C for 4 weeks, their SEC purities can be sequenced were ranked as follows: the protein solution having the combination numbered 6 > the protein solution having the combination numbered 9 > the protein solution having the combination numbered 1 > the protein solution having the combination numbered 3> the protein solution having the combination numbered 4 > the protein solution having the combination numbered 2 > the protein solution having the combination numbered 7. With regard to the degree of decrease in SEC purity, the protein solution having the combination of components numbered 6 showed a relatively smaller degree of decrease, while all protein solutions having other combinations of components showed similar degrees of decrease (maintained at about 1.5%) which were still acceptable.

**Table 8: CE-SDS purity**

| Number | Type of CE-SDS | CE-SDS purity | | | |
|---|---|---|---|---|---|
| | | 0 h | 4 °C 2 W | 40 °C 2 W | 40°C 4 W |
| 1 | Non-reducing | 100.0 | 100.0 | 99.0 | 92.4 |
| | Reducing (HC+LC) | HC+LC=99.4 | HC+LC=99.4 | HC+LC=98.5 | HC+LC=97.3 |
| | | NGHC=0.7 | NGHC=0.6 | NGHC=0.4 | NGHC=0.3 |
| 2 | Non-reducing | 100.0 | 99.7 | 99.4 | 94.9 |
| | Reducing (HC+LC) | HC+LC=99.3 | HC+LC=99.4 | HC+LC=98.3 | HC+LC=97.5 |
| | | NGHC=0.7 | NGHC=0.6 | NGHC=0.4 | NGHC=0.5 |
| 3 | Non-reducing | 100.0 | 100.0 | 99.4 | 94.8 |
| | Reducing (HC+LC) | HC+LC=99.3 | HC+LC=99.4 | HC+LC=98.2 | HC+LC=97.8 |
| | | NGHC=0.7 | NGHC=0.6 | NGHC=0.5 | NGHC=0.6 |
| 4 | Non-reducing | 100.0 | 100.0 | 99.5 | 95.4 |
| | Reducing (HC+LC) | HC+LC=99.3 | HC+LC=99.4 | HC+LC=98.4 | HC+LC=97.5 |
| | | NGHC=0.7 | NGHC=0.6 | NGHC=0.5 | NGHC=0.6 |
| 5 | Non-reducing | 100.0 | 100.0 | 98.7 | 94.9 |
| | Reducing (HC+LC) | HC+LC=99.4 | HC+LC=99.4 | HC+LC=98.5 | HC+LC=97.1 |
| | | NGHC=0.6 | NGHC=0.6 | NGHC=0.5 | NGHC=0.6 |
| 6 | Non-reducing | 100.0 | 100.0 | 99.6 | 94.5 |
| | Reducing (HC+LC) | HC+LC=99.4 | HC+LC=99.4 | HC+LC=99.2 | HC+LC=97.1 |
| | | NGHC=0.6 | NGHC=0.6 | NGHC=0.5 | NGHC=0.5 |
| 7 | Non-reducing | 100.0 | 99.9 | 97.9 | 89.9 |
| | Reducing (HC+LC) | HC+LC=99.3 | HC+LC=99.4 | HC+LC=98.0 | HC+LC=96.6 |
| | | NGHC=0.7 | NGHC=0.6 | NGHC=0.5 | NGHC=0.4 |
| 8 | Non-reducing | 100.0 | 99.9 | 95.9 | 88.1 |
| | Reducing (HC+LC) | HC+LC=99.4 | HC+LC=99.4 | HC+LC=97.7 | HC+LC=96.6 |
| | | NGHC=0.6 | NGHC=0.6 | NGHC=0.4 | NGHC=0.0 |
| 9 | Non-reducing | 100.0 | 100.0 | 99.5 | 97.0 |
| | Reducing (HC+LC) | HC+LC=99.4 | HC+LC=99.4 | HC+LC=98.8 | HC+LC=98.2 |
| | | NGHC=0.6 | NGHC=0.6 | NGHC=0.6 | NGHC=0.8 |

The data in Table 8 showed that among the protein solutions having the 9 combinations of components respectively which had been stored at 4 °C for 2 weeks, the lowest nrCE-SDS purity was 99.7%. In other words, all the protein solutions having the 9 combinations of components maintained the protein therein stable at 4 °C for 2 weeks. In addition, the values of HC+LC (rCE-SDS) after a storage at 4 °C for 2 weeks were little changed compared with those at 0 h respectively, namely all protein solutions having the combinations of components numbered 1 to 9 were relatively stable at 4 °C_{∘}

After being stored at 40 °C for 4 weeks, the protein solutions having the combinations of components numbered 7 and 8 showed significant decreases in nrCE-SDS purity (compared to that at 0 h respectively) and thus had low nrCE-SDS purities which were not acceptable. Further, except for the protein solution having the combination of components numbered 1 which had a nrCE-SDS purity of 92.4%, all other protein solution had nrCE-SDS purities greater than 94.0% and exhibited degrees of decrease (compared to that at 0 h respectively) of about 5%, which were acceptable.

Meanwhile, after being stored at 40°C for 4 weeks, the rCE-SDS purities of the protein solutions having different combinations of components were ranked as follows: the protein solution having the combination numbered 9 > the protein solution having the combination numbered 3 > the protein solution having the combination numbered 2 = the protein solution having the combination numbered 4 > the protein solution having the combination numbered 1 > the protein solution having the combination numbered 6 = the protein solution having the combination numbered 5 > the protein solution having the combination numbered 7 = the protein solution having the combination numbered 8. It can be seen the protein solutions having the combination of components numbered 7 and 8 exhibited the lowest purity, i.e., 96.6%, and each had a degree of decrease of about 3% (compared to that at 0 h respectively), larger than protein solutions having other combinations of components. rCE-SDS purities of all protein solutions having the combinations of components numbered 1 to 9 following a storage at 40 °C for 4 weeks were acceptable.

**Table 9: Charge heterogeneity**

| Number | Charge characteristics | Charge heterogeneity (proportion % of acid, neutral, and alkaline peaks) | | | |
|---|---|---|---|---|---|
| | | 0 h | 4 ° C2W | 40 ° C2 W | 40 ° C4 W |
| 1 | acid | 9.7 | 10.0 | 8.0 | 25.3 |
| | neutral | 89.9 | 89.0 | 90.0 | 69.9 |
| | alkaline | 0.5 | 1.1 | 2.0 | 4.8 |
| 2 | acid | 9.8 | 10.0 | 7.6 | 25.0 |
| | neutral | 89.4 | 89.5 | 89.6 | 71.1 |
| | alkaline | 0.8 | 0.5 | 2.9 | 3.9 |
| 3 | acid | 10.1 | 10.1 | 8.0 | 26.3 |
| | neutral | 89.5 | 89.2 | 90.0 | 70.2 |
| | alkaline | 0.4 | 0.7 | 2.0 | 3.5 |
| 4 | acid | 10.2 | 10.1 | 7.9 | 24.7 |
| | neutral | 88.6 | 89.3 | 89.5 | 68.2 |
| | alkaline | 1.2 | 0.5 | 0.5 | 7.1 |
| 5 | acid | 10.8 | 10.5 | 12.4 | 25.8 |
| | neutral | 87.4 | 88.5 | 85.9 | 69.9 |
| | alkaline | 1.8 | 0.9 | 1.7 | 4.3 |
| 6 | acid | 10.2 | 10.0 | 7.9 | 25.7 |
| | neutral | 87.2 | 88.7 | 90.8 | 70.2 |
| | alkaline | 2.6 | 1.3 | 1.3 | 4.1 |
| 7 | acid | 10.1 | 9.8 | 8.0 | 21.7 |
| | neutral | 88.4 | 89.9 | 88.3 | 67.3 |
| | alkaline | 1.5 | 0.2 | 3.6 | 11.0 |
| 8 | acid | 10.4 | 9.9 | 7.2 | 28.7 |
| | neutral | 88.8 | 88.6 | 87.0 | 69.8 |
| | alkaline | 0.7 | 1.4 | 5.8 | 1.5 |
| 9 | acid | 10.3 | 10.6 | 11.9 | 32.4 |
| | neutral | 88.4 | 88.9 | 86.8 | 63.3 |
| | alkaline | 1.3 | 0.5 | 1.4 | 4.3 |

The data in Table 9 showed that the protein solutions having the combinations of components numbered 1 to 9 exhibited different degrees of decrease and increase in the proportions of CEX neutral peak (compared to that at 0 h respectively) after being stored at 4 °C for 2 weeks. Among the protein solutions which showed decreases, the combination of components numbered 1 exhibited the largest decrease of 1%, and the decreased proportion of CEX neutral peak was still 89.0% which was acceptable. On the other hand, even the protein solution having the combination of components numbered 5 exhibited the lowest proportion of CEX neutral peak of 88.5% following a storage at 4 °C for 2 weeks, the proportion of CEX neutral peak was increased when compared to that at 0 h, and was still acceptable.

The proportions of CEX neutral peak in all the 9 protein solutions having the combinations of components decreased significantly following the storage at 40 °C for 4 weeks, and the decreased proportions were basically equal and most of them maintained in a range of 70%±2%, and were ranked as follows: the protein solution having the combination numbered 2 > the protein solution having the combination numbered 3 = the protein solution having the combination numbered 6 > the protein solution having the combination numbered 1 = the protein solution having the combination numbered 5 > the protein solution having the combination numbered 8 > the protein solution having the combination numbered 4 > the protein solution having the combination numbered 9. The largest degree of decrease was observed for the protein solution having the combination of components numbered 9, and compared to the proportion of CEX neutral peak after the storage, the percentage of the degree of decrease of the protein solution having the combination of components numbered 9 was 39.7%, and the corresponding percentages of the protein solutions having other combinations of components maintained in a range of 30%±5%.

**Table 10: DSC**

| Number | Tm1 | Tm2 |
|---|---|---|
| 1 | 63.90327 | 74.50385 |
| 2 | 64.63635 | 74.06069 |
| 3 | 64.34447 | 75.93933 |
| 4 | 64.73612 | 74.34037 |
| 5 | 65.15704 | 74.74829 |
| 6 | 64.65687 | 74.68807 |
| 7 | -- | 73.46957 |
| 8 | 62.28054 | 74.11718 |
| 9 | 62.21537 | 73.47579 |

The data in Table 10 showed that, with the exception of the protein solution having the combination of components numbered 7 which had only one Tm value (having no significant difference in comparison with other 8 protein solutions) obtained, the Tm1 and Tm2 values of the other protein solutions were all acceptable, and there was no significant difference between them.

**Table 11: Viscosity and osmotic pressure (0 h)**

| Number | Viscosity (cP) / temperature (°C) | Osmotic pressure (Osmol/kg) |
|---|---|---|
| 1 | 7.96/24.5 | 0.301/0.299/0.299 |
| 2 | 10.73/25.2 | 0.279/0.278 |
| 3 | 25.64/25.1 | 0.290/0.290 |
| 4 | 15.27/25.4 | 0.297/0.304/0.298 |
| 5 | 14.33/24.7 | 0.291/0.309/0.297 |
| 6 | 11.69/24.9 | 0.323/0.322 |
| 7 | 15.20/25.0 | 0.311/0.313 |
| 8 | 11.16/25.5 | 0.319/0.321 |
| 9 | 17.47/25.2 | 0.298/0.289 |

The data in Table 11 showed different degrees of decrease in viscosity of all protein solutions having the combinations of components numbered 1 to 9, and the viscosities of the solutions were ranked as follows: the protein solution having the combination numbered 1 < the protein solution having the combination numbered 2 < the protein solution having the combination numbered 8 < the protein solution having the combination numbered 6 < the protein solution having the combination numbered 5 < the protein solution having the combination numbered 7 < the protein solution having the combination numbered 4 < that numbered 9 < the protein solution having the combination numbered 3. Further, a comparison between the protein solutions having the combinations of components numbered 3, 4 and 5 showed that Tween 80 could reduce the viscosity of the protein solutions, but the degree of decrease was not linearly proportional to the concentration of Tween 80. For example, the protein solution comprising 0.2% Tween 80 showed a viscosity about 0.94 cP (6.2%) lower than the protein solution comprising 0.02% Tween 80. Comparison between the protein solutions having the combinations of components numbered 4, 7 and 9 showed that the protein solutions having combinations of components numbered 4 and 7 had similar viscosity, and the protein solution having the combination of components numbered 9 showed a viscosity 2.27 cP (13.0%) higher than the protein solution having the combination of components numbered 7, and 2.20 cP (12.6%) higher than the protein solution having the combination of components numbered 4, indicating pH 6.0 and pH 6.2 did a better job on reducing viscosity of the protein solution than pH 6.4, and pH 6.2 was a reasonable choice. Comparison between the protein solutions having the combinations of components numbered 1, 2 and 4 showed that the protein solution having the combination of components numbered 4 exhibited a viscosity 29.7% higher than the protein solution having the combination of components numbered 2, and a viscosity 48.8% higher than the protein solution having the combination of components numbered 1, indicating NaCl had a better effect on reducing viscosity of the protein solution than Tre. Additionally, the protein in the protein solution which only contained NaCl as a protective agent was hard to be stabilized, and thus a combination of NaCl and Tre at a proper ratio is required.

### Example 5: Effect of trehalose on the viscosity of the formulation

### Viscosity test (1) of the formulation:

A protein solution comprising CBP-201 antibody in a concentration of 102.8 mg/ml, 10 mmol/L His-HCl buffer (pH 6.2), and 40 mmol/L NaCl and no trehalose was prepared, and then concentrated with an ultra-centrifugal filter to a protein concentration of 133.6 mg/ml, 151.4 mg/ml, and > 151.4 mg/ml respectively. Viscosities of the concentrated protein solutions were tested, and then compared with the viscosity of the protein solution containing Tre. The results were shown in Table 12.

**Table 12: Detection results of the viscosity**

| Number | Target concentration of the protein (mg/ml) | Actual concentration of the protein (mg/ml) | Viscosity (cP) / temperature (°C) |
|---|---|---|---|
| 1 | 133.6 | 137.23 | 19.71/24.5 |
| 2 | 151.4 | 156.85 | 23.07/24.9 |
| 3 | > 151.4 | 173.36 | 33.88/25.2 |

The data in Table 12 showed that the viscosity increased as the increase in the concentration of the protein, and attention must be paid on the degree of over-concentration in advance when the protein solution was over-concentrated. A comparison between the protein solution in a concentration of 137.23 mg/ml which had a viscosity of 19.71 cP (24.5 °C) and the protein solution having the combination of components numbered 3 in Example 4 (which additionally contained Tre compared to the protein solution in this example and had a viscosity of 25.64 cP (25.1 °C)) indicated that Tre could increase the viscosity of the protein solution having said composition.

### Viscosity test (2) of the formulation:

A protein solution comprising CBP-201 antibody was prepared and then dialyzed in a dialysis buffer (10 mmol/L His-HCl, pH 6.2, 40 mmol/L NaCl) supplemented with 150 mmol/L trehalose (1×) by tangential flow and concentrated. SEC purity and viscosity were detected and the results were shown in Table 13.

**Table 13: Detection results of the viscosity**

| Number | Actual concentration of the protein (mg/ml) | Viscosity (cP) / temperature (°C) |
|---|---|---|
| 1 | 53.69 | 3.93/25.2 |
| 2 | 133.37 | 23.18/25.0 |
| 3 | 155.15 | 45.22/24.9 |

The results of SEC purity showed that SEC purity did not significantly decrease during the dialysis and concentration, indicating that the process and the way for the dialysis and concentration did not affect the SEC purity of the protein.

The detection results of the viscosity showed that, the viscosity of the protein solution having a protein concentration of 133.37 mg/ml was 23.18 cP (25.0 °C), which was basically consistent with the results obtained from the protein solution having the combination of components numbered 3 in Example 4 (which had a viscosity of 25.64 cP (25.1 °C)), indicating the present technical solution of the invention has repeatability. A viscosity of 45.22 cP (24.9 °C) was shown by the protein solution having a protein concentration of 155.15 mg/ml, which was 49.0% higher than the viscosity shown by the protein solution having a protein concentration of 156.85 mg/ml in Viscosity test (1) (23.07 cP (24.9 °C)). Obviously, the addition of Tre increased the viscosity of the protein solution, and it should be added appropriately under the premise of ensuring the stability of the protein.

### Example 6: Production of CBP-201 antibody formulation

### Clarification by filtration

Debris in cell culture was removed through a step of clarification by filtration firstly. In the process of the clarification by filtration, MD0HC pod filter and MA1HC pod filter from Millipore Corporation were used in combination. The filters were installed and fixed to pod holders, and then rinsed with water for injection and PBS respectively. When the rinsing was completed, liquid at the inlet was switched to the cell culture, and the inlet pressure was set to 0-20 psi. The pod filters were washed with PBS when feeding was finished, and all the liquid vented was harvested.

### Affinity chromatography

A chromatographic column was filled with MabSelect SuRe LX resin to obtain a column height of 18-22 cm, and a symmetry factor of 0.8-1.0 and number of theoretical plates ≥ 2000 N/m were ensured. The column was equilibrated with 4-6 column volumes of an equilibration buffer (0.025 mol/L Tris, 0.10 mol/L NaCl, pH7.40 ± 0.20) for affinity chromatography, and the liquid harvested during the clarification by filtration was loaded thereon. The column was further equilibrated with 3-4 column volumes of the equilibration buffer for affinity chromatography after the loading. Afterwards, the column was rinsed with 2-3 column volumes of a rinsing solution 1 (0.025 mol/L Tris, 1.00 mol/L NaCl, pH7.40 ± 0.20) and a rinsing solution 2 (0.05 mol/L NaAc, pH 5.50 ± 0.10) respectively. When the rinsing was completed, the protein was eluted with an eluent (0.10 mol/L NaAc-HAc, pH 3.60 ± 0.10) for affinity chromatography. The collection of the protein was started when the UV absorption value rose to 0.1000-0.1500 AU/5mm, and was stopped when the UV absorption value dropped to 0.2000-0.3000 AU/5mm.

### Virus inactivation at low pH

An acidic titrant (1.00 mol/L acetic acid) was prepared and used to adjust the pH of the protein solution eluted through the affinity chromatography to 3.50-3.70, and the obtained protein solution was placed at 20-25 °C for 2-3 h for virus inactivation at low pH. After that, the pH of the protein solution was adjusted to 5.40-5.60 using a basic titrant (2.00 mol/L Tris) and the conductivity of the protein solution was detected. By using a sterilizing filter, the protein solution was filtered into a disposable liquid container, and then sampled for protein concentration, pH, conductivity, bacterial endotoxin, SEC-HPLC, CEX-HPLC, CE-SDS, and microbial limit tests.

### Cation-exchange chromatography

In the cation-exchange chromatography, Capto S ImpAct resin, a column height of 18-22 cm, a symmetry factor of 0.8-1.8, and number of theoretical plates ≥ 2000 N/m were employed. The column was equilibrated with 4-6 column volumes of an equilibration buffer (0.05 mol/L NaAc, 0.05 mol/L NaCl, pH 5.50 ± 0.05) for cation-exchange chromatography, followed by loading the protein solution thereon. The column was further equilibrated with 2-4 column volumes of the equilibration buffer for cation-exchange chromatography after the loading. Afterwards, the column was rinsed with 5-7 column volumes of a rinsing solution (0.05 mol/L NaAc, 0.08 mol/L NaCl, pH 5.50 ± 0.05) for cation-exchange chromatography . When the rinsing was completed, the protein was eluted with an eluent (0.05 mol/L NaAc, 0.22 mol/L NaCl, pH 5.50 ± 0.05) for cation-exchange chromatography. The collection of the protein was started when the UV absorption value rose to 0.2000-0.3000 AU/5mm, and was stopped when the UV absorption value dropped to 1.0000-1.5000 AU/5mm.

### Anion-exchange chromatography

In the anion-exchange chromatography, POROS 50 HQ resin, a column height of 18-22 cm, a symmetry factor of 0.8-1.8, and number of theoretical plates ≥ 2000 N/m were employed. The pH of the protein solution was adjusted to 7.35-7.45 before loading it on the column, and then the conductivity of the protein solution was detected. By using a sterilizing filter (load capacity ≤ 3000 g/m²), the protein solution was then filtered into a disposable liquid container.

### Nanofiltration

A nanofiltration system was assembled and the prefiltration membrane was rinsed with the equilibration buffer for anion-exchange chromatography. When the rinsing of the membrane was completed, a sterilizing filter was connected to the nanofiltration system and the nanofiltration of the protein was conducted under a pressure of 28-32 psi for no longer than 4 h.

### Concentration by dialyzing

Sartocon cassette 30KD was used. Firstly, the cassette was assembled to an ultrafiltration system and integrity and water flux were tested. The membrane in the cassette was then rinsed with a dialysis buffer (0.060 mol/L trehalose, 0.010 mol/L histidine-HCl, 0.10 mol/L NaCl, pH 6.20 ± 0.05) if the tests were passed. After the rising, the nano-membrane filtrated protein solution obtained as above described was stirred to mix well and then loaded onto the membrane and pre-concentrated to 40-60 mg/mL. After the pre-concentration, dialysis was conducted with the dialysis buffer in a volume 10-12 times the volume of the pre-concentrated protein solution. The protein solution was then over-concentrated to obtain a protein concentration of 170-190 mg/mL. The membrane was rinsed with the dialysis buffer 2 times and liquid obtained from the rinsing was collected. The volume of the dialysis buffer each time used for rinsing the membrane was about 1.5 times the system hold-up volume, and the total volume used for rinsing the membrane was about 2-4 times the system hold-up volume. Finally, the protein concentration of the solution was adjusted to 140-180 mg/mL, and the final protein concentration was detected.

### Preparation of a stock solution (Dilution and excipient addition)

The protein solution was diluted with a Tween 80 stock solution (2% Tween (w/v)) and a dialysis buffer (0.060 mol/L trehalose, 0.010 mol/L histidine-HCl, 0.10 mol/L NaCl, pH 6.20 ± 0.05) to 150 ± 5 mg/mL, and the finial concentration of Tween 80 therein was 0.02%. The diluted protein solution was then filtered into a disposable liquid container, and the obtained protein solution which had been filtered was the stock solution. The prepared stock solution was divided into PETG bottles and stored at -80 ± 10 °C.

### Preparation of a formulation

The stock solution was taken, thawed at room temperature, mixed well, and then filtrated. Rubber stoppers and alumunium covers were packed and sterilized, and vials which had been cleaned to remove pyrogens were transferred to corresponding sites for filling. The filled vials which had been all stoppered were then transferred to a capping room for capping. After that, visual inspection was performed vial by vial to eliminate ones which had defects such as inaccurate loading volume, collapse, visible particles, foreign matters, broken cap and empty vials. Then the vials were labeled and packed in boxes, and then at the center of the upper surfaces of the boxes a carton label was pasted.

Acceptance criteria and detection results were shown in Table 14.

**Table 14: Acceptance criteria and detection results**

| **Item examined** | **Acceptance Criteria** | **The first batch** | **The second batch** |
|---|---|---|---|
| Appearance/Color | Colorless to pale yellow solution, not darker | Colorless and clear solution | pale yellow solution, lighter than Y4 |
| | than Y4 standard colorimetric solution | | standard colorimetric solution |
| Clarity | TBD | 26.4 NTU | 20.8 NTU |
| Identification (nrCE-SDS) | The pattern should be consistent with the reference | The pattern was consistent with the reference | The pattern was consistent with the reference |
| Identification (rCE-SDS) | The pattern should be consistent with the reference | The pattern was consistent with the reference | The pattern was consistent with the reference |
| Identification (pl-iCIEF) | The main peak pl is 7.6-8.6, and the pattern should be consistent with the reference. (The difference from the main peak pl of the reference is not more than ±0.200) | PI = 8.2, and the pattern was consistent with the reference. | The main peak pl was 8.1, and the pattern was consistent with the reference. (0.015 difference from the reference pl) |
| Insoluble particles | Particles having a particle size of ≥ 10 µm shall not exceed 6000 / vial; | ≥ 10 µm: 10.0 particles /vial; | ≥ 10µm: 35 particles /vial; |
| | Particles having a particle size of ≥ 25 µm shall not exceed 600 / vial | ≥ 25 µm: 0.7 particles /vial | ≥ 25µm: 8 particles /vial |
| Visible foreign matter | No obvious visible foreign matter | No fine visible foreign matter | No obvious visible foreign matter |
| Impurity (aggregates) (SEC-HPLC) | HWM% ≤ 10.0% | 0.4% | 2.2% |
| CEX purity | TBD | Acid peak = 8.5% | Acid peak = 13.4% |
| | | Alkaline peak = 1.7% | Alkaline peak = 1.7% |
| | | Neutral peak = 89.8% | Neutral peak = 84.9% |
| Purity (nrCE-SDS) | Main peak proportion should ≥ 90.0% | 97.6% | 95.6% |
| Purity (rCE-SDS) | HC+LC ≥ 95.0% | HC+LC = 99.1% | HC+LC = 98.9% |
| Content of Tween 80 | TBD | 0.19 mg/mL | 0.13 mg/mL |
| Purity (aggregates + degradation products) (SEC-HPLC) | Main peak proportion should ≥ 90.0% | 99.6% | 97.8% |
| Binding activity | Relative binding activity is 50% - 150% | 99.3% | 106% |
| Biological activity | Relative cell activity is 50% - 150% | 108.3% | 107.1% |
| Protein concentration | 126.0-154.0 mg/mL | 140.4 mg/mL | 141.3 mg/mL |
| pH value | 5.9-6.5 | 6.2 | 6.2 |
| fill volume | Not less than 1 mL | Fill volume of the vials tested: 1.16 mL; 1.17 mL; 1.17 mL, 1.16 mL; 1.16 mL | Maximum fill volume: 1 mL; |
| | | | Minimum fill volume: 1 mL; |
| | | | Average fill volume: 1 mL |
| Osmotic pressure molar concentration | 230-330 mOsmol/kg | 290 mOsmol/kg | 286 mOsmol/kg |
| Sterility | There should be no bacterial growth | No bacterial growth | No bacterial growth |
| Bacterial endotoxin | < 0.3 EU/mg protein | < 0.1 EU/mg protein | < 0.3 EU/mg protein |

### Example 7: Detection on binding activity of the CBP-201 antibody formulation

Indirect ELISA was used to detect the binding ability of the present formulation to the antigen sIL-4Rα.

The antigen (sIL-4Rα, expressed according to NM_000418.4) was coated and absorbed on a solid phase plate for ELISA, and then plate was washed, blocked and washed again before adding the samples to be tested for binding with the antigen. The samples to be tested were dilutions of the formulation prepared according to Example 6 of the present application, and the dilutions were obtained by diluting the formulation with 1% BSA to an antibody concentration of 1000 ng/mL, and subsequently performing a gradient dilution from an initial concentration of 1000 ng/mL to a concentration of 0 ng/mL. The unbound antibody was removed through washing after incubation, and an enzyme labeled secondary antibody was added for further incubation. After the unbound enzyme labeled secondary antibody was removed through washing, enzyme reaction substrate was added for color developing. Finally, the reaction was stopped by adding a stop solution, and absorbance values were read at 450 nm and 655 nm on a microplate reader. According to the absorbance values, half effective concentration EC₅₀ was calculated by curve fitting.

Detection results of the binding activity of the CBP-201 formulation were shown in Table 15.

**Table 15: Detection results of the binding activity**

| Number | The first batch of the CBP-201 formulation EC₅₀ (ng/ml) | The second batch of the CBP-201 formulation EC₅₀ (ng/ml) |
|---|---|---|
| 1 | 1.587 | 1.400 |
| 2 | 1.516 | 1.282 |
| 3 | 1.837 | 1.767 |
| 4 | 1.849 | 1.801 |
| 5 | 1.837 | 1.767 |
| 6 | 1.533 | 1.446 |
| Average value | 1.693 | 1.557 |

**Example 8:** Detection on biological activity of the CBP-201 antibody formulation HEK Blue^{™} IL4/IL13 cells (available from Invivogen) were used to detect activity of the CBP-201 formulation on blocking STAT-6 signal transduction.

HEK Blue^{™} IL4/IL13 cells were plated on a 384-well cell culture plate and the samples to be tested were added therein. The samples to be tested were dilutions of the formulation prepared according to Example 6 of the present application, and the dilutions were obtained by diluting the formulation with DMEM containing 10% FBS to an antibody concentration of 5000 ng/mL, and subsequently performing a gradient dilution from an initial concentration of 5000 ng/mL to a concentration of 0 ng/mL. Afterwards, IL4 (available from Invivogen) was added to the plate to obtain a final concentration of 0.5 ng/mL. After incubating in a cell incubator for 22 h, supernatant was taken and Quanti-blue was added therein for color developing, then absorbance values were read at 650 nm on a microplate reader. According to the absorbance values, half inhibitory concentration IC₅₀ was calculated by curve fitting.

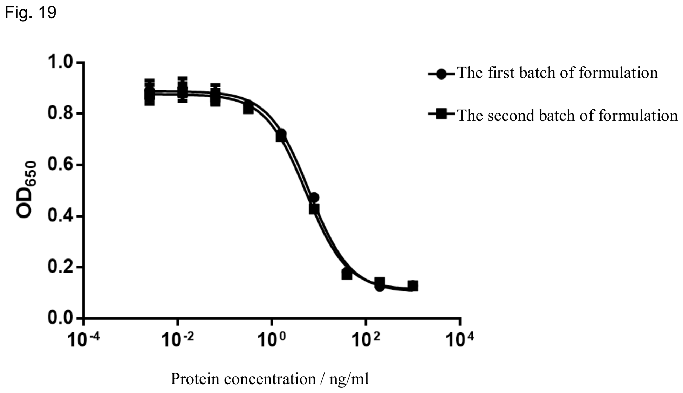

Detection results of the biological activity of the CBP-201 formulation were shown in Table 16 and Figure 19.

**Table 16: Detection results of the biological activity**

| Number | The first batch of the CBP-201 formulation | The second batch of the CBP-201 formulation |
|---|---|---|
| | IC50 (ng/ml) | IC50 (ng/ml) |
| 1 | 3.283 | 3.339 |
| 2 | 3.637 | 4.276 |
| 3 | 3.373 | 4.657 |
| 4 | 5.379 | 6.339 |
| 5 | 4.154 | 4.062 |
| 6 | 5.331 | 4.407 |
| Average value | 4.193 | 4.513 |

The activity of the prepared CBP-201 formulation was detected using HEK Blue^{™} IL4/IL13 cells, and average values of IC50 of the two batches were 4.193 ng/ml and 4.513 ng/ml respectively.

The above description for the embodiments of the present invention is not intended to limit the present invention, and those skilled in the art can make various changes and variations according to the present invention, which are within the protection scope of the claims of the present invention without departing from the spirit of the same.

### ITEMS

1. A liquid composition comprising an antibody against human interleukin-4 receptor alpha, wherein the liquid composition comprises the antibody at a concentration of 50-200 mg/ml; and a buffer, a protective agent and a surfactant which serve as excipients, and the liquid composition has a pH of 5.4-6.4.
2. The liquid composition according to Item 1, wherein in the liquid composition, the antibody comprises a light chain variable region and a heavy chain variable region, and wherein the light chain variable region comprises CDR1 comprising the amino acid sequence as set forth in SEQ ID NO: 1, CDR2 comprising the amino acid sequence as set forth in SEQ ID NO: 2, and CDR3 comprising the amino acid sequence as set forth in SEQ ID NO: 3, and the heavy variable region comprises CDR1 comprising the amino acid sequence as set forth in SEQ ID NO: 5, CDR2 comprising the amino acid sequence as set forth in SEQ ID NO: 6, and CDR3 comprising the amino acid sequence as set forth in SEQ ID NO: 7;
   preferably, the antibody comprises a light chain variable region comprising the amino acid sequence as set forth in SEQ ID NO: 4, and a heavy variable region comprising the amino acid sequence as set forth in SEQ ID NO: 8;
   preferably, the antibody is present at a concentration of 100-200 mg/ml, more preferably 130-165 mg/ml, and further preferably 150±5 mg/ml.
3. The liquid composition according to Item 1 or 2, wherein the buffer is one or more selected from the group consisting of an acetate buffer, a phosphate buffer and an amino acid buffer, and the buffer is present at a concentration of 5-50 mmol/L; preferably, the buffer is an amino acid buffer at a concentration of 5-50 mmol/L.
4. The liquid composition according to Item 3, wherein the acetate buffer is sodium acetate buffer, the phosphate buffer is sodium dihydrogen phosphate buffer, or the amino acid buffer is histidine hydrochloride buffer;
   preferably, the buffer is present at a concentration of 5-20 mmol/L;
   preferably, the buffer is histidine hydrochloride buffer at a concentration of 5-20 mmol/L, more preferably histidine hydrochloride buffer at a concentration of 10 mmol/L.
5. The liquid composition according to any one of Items 1 to 4, wherein the protective agent is one or more selected from the group consisting of a sugar, an alcohol, an amino acid and a chloride salt, and the protective agent is present at a concentration of 40-220 mmol/L.
6. The liquid composition according to Item 5, wherein the protective agent is one or more selected from the group consisting of a sugar, an alcohol and an amino acid, and the protective agent is present at a concentration of 40-220 mmol/L, and/or the protective agent is a chloride salt, and the protective agent is present at a concentration of 40-150 mmol/L;
   preferably, the sugar is trehalose and/or sucrose at a concentration of 40-150 mmol/L, more preferably 60-150 mmol/L; the alcohol is mannitol at a concentration of 40-220 mmol/L, more preferably 110-150 mmol/L; the amino acid is one or more selected from the group consisting of proline, arginine hydrochloride and glycine at a concentration of 40-220 mmol/L, more preferably 120-220 mmol/L; or the chloride salt is sodium chloride at a concentration of 40-150 mmol/L, more preferably 80-120 mmol/L;
   preferably, the protective agent is a combination of trehalose and sodium chloride; more preferably, the protective agent is a combination of 40-150 mmol/L trehalose and 40-150 mmol/L sodium chloride; further preferably, the protective agent in the liquid composition is a combination of 60-150 mmol/L trehalose and 80-120 mmol/L sodium chloride, more preferably a combination of 60 mmol/L trehalose and 100 mmol/L sodium chloride.
7. The liquid composition according to any one of Items 1 to 6, wherein the surfactant is a non-ionic polymer such as is one or more selected from the group consisting of Tween 80, Tween 20, Poloxamer and polyethylene glycol, and the surfactant is present at a concentration of 0.01%-0.2%;
   preferably, the surfactant is 0.01-0.03% Tween 80, more preferably 0.02% Tween 80.
8. The liquid composition according to any one of Items 1 to 7, wherein the liquid composition is a formulation for injection, and preferably for subcutaneous or intravenous injection;
   preferably, the liquid composition comprises:
   130-165 mg/ml, preferably 150±5 mg/ml of the antibody;
   10 mmol/L histidine hydrochloride;
   60 mmol/L trehalose;
   100 mmol/L sodium chloride;
   0.02% Tween 80; and
   the liquid composition has a pH of 6.2±0.2, preferably 6.2±0.05.
9. Use of the liquid composition according to any one of Items 1 to 8 for manufacturing a medicament for the treatment of inflammation or allergic disease;
   preferably, the inflammation or allergic disease includes autoimmune disease, such as allergic dermatitis, asthma, eosinophilic esophagitis, eczema, allergic rhinitis, nasal polyp, rheumatoid arthritis, and the like.
10. A container or a kit comprising the container, wherein the container comprises the liquid composition according to any one of Items 1 to 8.
11. A method of preventing, treating or ameliorating inflammation or allergic disease, wherein the method includes administering to a subject in need thereof the liquid composition according to any one of Items 1 to 8;
   preferably, the subject is a mammal, more preferably, a human;
   preferably, the inflammation or allergic disease includes autoimmune disease, such as allergic dermatitis, asthma, eosinophilic esophagitis, eczema, allergic rhinitis, nasal polyp, rheumatoid arthritis, and the like;
   preferably, the liquid composition is administrated to the subject by injection, for example by subcutaneous injection or intravenous injection.

## Claims

1. A liquid composition comprising an antibody against human interleukin-4 receptor alpha, wherein the liquid composition comprises the antibody at a concentration of 50-200 mg/ml; and a buffer, a protective agent and a surfactant which serve as excipients, and the liquid composition has a pH of 5.4-6.4.

2. The liquid composition according to Claim 1, wherein in the liquid composition, the antibody comprises a light chain variable region and a heavy chain variable region, and wherein the light chain variable region comprises CDR1 comprising the amino acid sequence as set forth in SEQ ID NO: 1, CDR2 comprising the amino acid sequence as set forth in SEQ ID NO: 2, and CDR3 comprising the amino acid sequence as set forth in SEQ ID NO: 3, and the heavy variable region comprises CDR1 comprising the amino acid sequence as set forth in SEQ ID NO: 5, CDR2 comprising the amino acid sequence as set forth in SEQ ID NO: 6, and CDR3 comprising the amino acid sequence as set forth in SEQ ID NO: 7.

3. The liquid composition according to Claim 1 or 2, wherein in the liquid composition, the antibody comprises a light chain variable region comprising the amino acid sequence as set forth in SEQ ID NO: 4, and a heavy variable region comprising the amino acid sequence as set forth in SEQ ID NO: 8.

4. The liquid composition according to any one of Claims 1 to 3, wherein the antibody is present at a concentration of 100-200 mg/ml, more preferably 130-165 mg/ml, and further preferably 150±5 mg/ml.

5. The liquid composition according to any one of Claims 1 to 4, wherein the buffer is one or more selected from the group consisting of an acetate buffer, a phosphate buffer and an amino acid buffer, and the buffer is present at a concentration of 5-50 mmol/L; preferably, the buffer is an amino acid buffer at a concentration of 5-50 mmol/L.

6. The liquid composition according to Claim 5, wherein the acetate buffer is sodium acetate buffer, the phosphate buffer is sodium dihydrogen phosphate buffer, or the amino acid buffer is histidine hydrochloride buffer;
wherein the buffer is present at a concentration of 5-20 mmol/L;
preferably, the buffer is histidine hydrochloride buffer at a concentration of 5-20 mmol/L, more preferably histidine hydrochloride buffer at a concentration of 10 mmol/L.

7. The liquid composition according to any one of Claims 1 to 6, wherein the protective agent is one or more selected from the group consisting of a sugar, an alcohol, an amino acid and a chloride salt, and the protective agent is present at a concentration of 40-220 mmol/L.

8. The liquid composition according to Claim 7, wherein the protective agent is one or more selected from the group consisting of a sugar, an alcohol and an amino acid, and the protective agent is present at a concentration of 40-220 mmol/L, and/or the protective agent is a chloride salt, and the protective agent is present at a concentration of 40-150 mmol/L.

9. The liquid composition according to Claim 7 or 8, wherein the sugar is trehalose and/or sucrose at a concentration of 40-150 mmol/L, preferably 60-150 mmol/L; the alcohol is mannitol at a concentration of 40-220 mmol/L, preferably 110-150 mmol/L; the amino acid is one or more selected from the group consisting of proline, arginine hydrochloride and glycine at a concentration of 40-220 mmol/L, preferably 120-220 mmol/L; or the chloride salt is sodium chloride at a concentration of 40-150 mmol/L, preferably 80-120 mmol/L.

10. The liquid composition according to any one of Claims 7 to 9, wherein the protective agent is a combination of trehalose and sodium chloride; preferably, the protective agent is a combination of 40-150 mmol/L trehalose and 40-150 mmol/L sodium chloride; further preferably, the protective agent in the liquid composition is a combination of 60-150 mmol/L trehalose and 80-120 mmol/L sodium chloride, preferably a combination of 60 mmol/L trehalose and 100 mmol/L sodium chloride.

11. The liquid composition according to any one of Claims 1 to 10, wherein the surfactant is a non-ionic polymer such as is one or more selected from the group consisting of Tween 80, Tween 20, Poloxamer and polyethylene glycol, and the surfactant is present at a concentration of 0.01%-0.2%;
preferably, the surfactant is 0.01-0.03% Tween 80, more preferably 0.02% Tween 80.

12. The liquid composition according to any one of Claims 1 to 11, wherein the liquid composition is a formulation for injection, and preferably for subcutaneous or intravenous injection.

13. The liquid composition according to any one of Claims 1 to 12, wherein the liquid composition comprises:
130-165 mg/ml, preferably 150+5 mg/ml of the antibody;
10 mmol/L histidine hydrochloride;
60 mmol/L trehalose;
100 mmol/L sodium chloride;
0.02% Tween 80; and
the liquid composition has a pH of 6.2±0.2, preferably 6.2±0.05.

14. A container or a kit comprising the container, wherein the container comprises the liquid composition according to any one of Claims 1 to 13.

15. The liquid composition according to any one of Claims 1 to 13 for use in method of preventing, treating or ameliorating inflammation or allergic disease, wherein the method includes administering to a subject in need thereof the liquid composition according to any one of Claims 1 to 13;
preferably, the subject is a mammal, more preferably, a human;
preferably, the inflammation or allergic disease includes autoimmune disease, such as allergic dermatitis, asthma, eosinophilic esophagitis, eczema, allergic rhinitis, nasal polyp, rheumatoid arthritis, and the like;
preferably, the liquid composition is administrated to the subject by injection, for example by subcutaneous injection or intravenous injection.
